# EUROPEAN PATENT APPLICATION

(11) **EP 4 582 546 A1**
(43) Date of publication of application: **09.07.2025**
(21) Application number: 23859432.9
(22) Date of filing: 31.08.2023
(51) Int. Cl.: C12N 15/864, C12N 15/86, C12N 15/35, A61K 35/76, A61K 48/00, C12R 1/93

(54) **ADENO-ASSOCIATED VIRUS STRUCTURAL PLASMID CAPABLE OF IMPROVING ADENO-ASSOCIATED VIRUS TITER**

(30) Priority: 31.08.2022 CN 202211056510
(71) Applicant: Zhenjiang Probio Biotech Co., Ltd., Zhenjiang, Jiangsu 212009 (CN)
(72) Inventor: GU, Lengtao, Zhenjiang, Jiangsu 212009 (CN); XUAN, Chunling, Zhenjiang, Jiangsu 212009 (CN); CHEN, Zhen, Zhenjiang, Jiangsu 212009 (CN)
(74) Representative: Brann AB
(86) International application number: PCT/CN2023/115991
(87) International publication number: WO 2024/046403

(57) **Abstract**

The present disclosure relates to the technical field of molecular biology, and in particular to an adeno-associated virus structural plasmid capable of improving adeno-associated virus titer, which is provided with a Rep gene expression cassette and a Cap gene expression cassette in sequence in the gene expression direction. The Rep gene expression cassette comprises a rep gene regulated and transcribed by a first promoter, and a transcription termination signal fragment is arranged behind the rep gene; the Cap gene expression cassette comprises a cap gene regulated and transcribed by a second promoter, wherein the first promoter comprises any one of a P5 promoter, an RSV promoter, an MMTV promoter, a UBC promoter, and a U6 promoter, and the second promoter comprises one or more of a CMV promoter, a CBh promoter, a CAG promoter, an EF1α promoter, and an SFFV promoter.

## Description

### Cross-Reference to Related Applications

The present application claims the priority of Chinese patent application no. 202211056510.1 filed on August 31, 2022, which is incorporated herein by reference in its entirety.

### Technical Field

The present disclosure relates to the technical field of molecular biology, and in particular to an adeno-associated virus structural plasmid capable of improving adeno-associated virus titer.

### Background Art

Gene therapy and cell therapy have entered a stage of rapid development and become the most promising development direction of medical care for life. The main goal of gene therapy is to introduce an exogenous gene into a target cell or tissue to replace, compensate for, block, and modify a specific gene, so as to achieve the purpose of treating a disease. In gene therapy, 70%-80% of therapeutic regimens are accomplished by virus vectors. The virus vector is a key vehicle to deliver a therapeutic exogenous gene to a target site, and can be used either as a drug for direct injection or as an important raw material for drug production. Virus vectors currently used for introducing exogenous genes into target cells are mainly derived from virus vectors such as a retrovirus vector, an adenovirus vector, and an adeno-associated virus vector.

Adeno-associated virus (AAV), a member of the family Parvoviridae, is a type of icosahedral parvovirus with a diameter of about 20-26 nm, which cannot replicate autonomously, has no envelope, and is composed of a protein capsid and a single-stranded DNA genome with a size of 4.7 kb. The protein capsid is composed of three subunits, i.e., VP1, VP2, and VP3, respectively. There are two "T"-shaped inverted terminal repeats (ITRs) at the two ends of the AAV genome. These two ITRs are the origin of replication of the viral DNA and a signal that triggers viral packaging. The *rep* gene in the AAV genome encodes four proteins related to virus replication, i.e., Rep78, Rep68, Rep52, and Rep40, respectively. The recombinant adeno-associated virus (rAAV) vector used in the study is a gene vector modified on the basis of non-pathogenic wild-type AAV. The rAAV is regarded as one of the most promising vectors for gene research and gene therapy due to its diverse types, extremely low immunogenicity, high safety, wide host cell range (infectivity to both dividing cells and non-dividing cells), strong diffusion ability, long time of gene expression *in vivo,* etc.

There are mainly three kinds of helper expression systems for producing adeno-associated virus vectors: adenovirus (AdV), herpesvirus (HSV), and baculovirus (BV) expression systems. Adenovirus helper system involves the transient transfection of three plasmids into HEK293 cells that constitutively express adenoviral *E1a* and *E1b* genes, wherein one plasmid expresses *rep* and *cap* genes of adeno-associated virus; another plasmid expresses a target gene that needs to be delivered *in vivo*; and yet another plasmid expresses adenovirus genes with helper function, thus producing an AAV. The herpesvirus helper system involves placing the AAV gene into HSV1 genome and using co-infection with HSV to produce an AAV. The baculovirus helper system exploits the ability of Sf9 cells to express recombinant proteins in large quantities, and by transducing Sf9 cells with an insect baculovirus containing rAAV genome and rAAV *rep*/*cap* genes, the AAV assembly in the Sf9 cells is enabled, ultimately achieving the purpose of large-scale production of AAVs. At present, most adeno-associated viruses are produced by transient transfection of three plasmids, followed by packaging into viruses. For enterprises engaged in research and development of cell and gene therapies, the quantity demanded for virus vectors is relatively low during preclinical research, IND application, and clinical trials. However, as the demand increases, a significant disparity emerges between the titer of virus vectors from production and the stringent requirements of gene therapy on the quality (including virus titer and residual substances) and quantity of virus vectors. Therefore, the availability of a virus vector that meets the ever-expanding clinical demand is still a big obstacle and one of the biggest limiting factors for potential commercial supply. At present, the yield of AAV production per unit from different platforms ranges from 10³ to 10⁵ vector genomes (vg) per cell, and global scientific researchers are making efforts to improve the vector yield, so as to meet the demand for a high yield. Generally, the best approach is to modify AAV-related genes to facilitate the production of safe and high-quality vectors.

### Summary of the Invention

A first object of the present disclosure is to provide an adeno-associated virus structural plasmid, which is provided with a Rep gene expression cassette and a Cap gene expression cassette in sequence in the gene expression direction, wherein
the Rep gene expression cassette comprises a *rep* gene regulated by a first promoter for transcription, followed by a transcription termination signal fragment arranged downstream of the *rep* gene; and the Cap gene expression cassette comprises a *cap* gene regulated by a second promoter for transcription,
wherein the first promoter comprises any one of a P5 promoter, an RSV promoter, an MMTV promoter, a UBC promoter, and a U6 promoter, and the second promoter comprises one or more of a CMV promoter, a CBh promoter, a CAG promoter, an EF1α promoter, and an SFFV promoter.

A second object of the present disclosure is to provide a kit comprising the adeno-associated virus structural plasmid as described above.

A third object of the present disclosure is to provide a method for preparing adeno-associated virus particles, comprising introducing the adeno-associated virus structural plasmid as described above, together with a transfer plasmid and a helper plasmid, into a host cell for packaging.

The adeno-associated virus structural plasmid can significantly improve the titer and infectious activity of adeno-associated viruses.

### Brief Description of the Drawings

In order to illustrate the technical solutions in the Detailed Description of Embodiments of the present disclosure or in the prior art more clearly, the accompanying drawings required in the description of the Detailed Description of Embodiments or the prior art are briefly described below. It is clear that the accompanying drawings in the following description illustrate some embodiments of the present disclosure, and those of ordinary skill in the art may further derive other drawings from these accompanying drawings without involving any inventive effort.
FIG. 1 is a map of plasmid 1#;
FIG. 2 is a map of plasmid 4#;
FIG. 3 shows electrophoregrams of synthesized plasmids 3#, 4#, and 5# after enzymatic digestion;
FIG. 4 shows electrophoregrams of amplified plasmids 3#, 4#, and 5# after enzymatic digestion;
FIG. 5 is a graph of viable cell density and cell viability after transfection with different structural plasmid systems;
FIG. 6 shows the AAV genome copy number after transfection with different structural plasmid systems;
FIG. 7 shows the AAV transduction titer after transfection with different structural plasmid systems;
FIG. 8 is a graph of viable cell density and cell viability after transfection with different plasmid systems having strong promoters;
FIG. 9 shows the AAV genome copy number after transfection with different plasmid systems having strong promoters; and
FIG. 10 shows the AAV transduction titer after transfection with different plasmid systems having strong promoters.

### Detailed Description of Embodiments

Reference will now be made in detail to the embodiments of the present disclosure, one or more examples of which are set forth below. Each example is provided as an explanation rather than a limitation of the present disclosure. Indeed, it would have been obvious to a person skilled in the art that various modifications and variations may be made to the present disclosure without departing from the scope or spirit of the present disclosure. For instance, features illustrated or described as part of one embodiment, can be used on another embodiment to yield a still further embodiment.

Unless otherwise stated, all terms (including technical and scientific terms) used to disclose the present disclosure have the same meaning as commonly understood by those of ordinary skill in the art to which the present disclosure belongs. With further guidance, ensuing definitions are used to better understand the teachings of the present disclosure. Herein, the terms used in the description of the present disclosure are merely for the purpose of describing specific examples, but are not intended to limit the present disclosure.

The terms "and/or" and "or/and" used herein are selected to encompass any one of two or more associated items listed therein, as well as any and all combinations of the associated items listed therein, wherein the combinations include combinations of any two of the associated items listed therein, any more of the associated items listed therein, or all of the associated items listed therein. It should be noted that when at least three items are connected by a combination of at least two conjunctions selected from "and/or" and "or/and", it should be understood that in the present application, the technical solutions definitely include the technical solution in which items are all connected by "logic AND" and also definitely include the technical solutions in which items are all connected by "logic OR". For example, "A and/or B" includes the three parallel solutions of A, B and A+B. As another example, the technical solution of "A, and/or, B, and/or, C, and/or, D" includes any one of A, B, C, and D (i.e., the technical solutions in which items are all connected by "logic OR"), also includes any and all combinations of A, B, C, and D, that is, combinations of any two or any three of A, B, C, and D, and further includes the combination of all the four items A, B, C, and D (i.e., the technical solution in which items are all connected by "logic AND").

The terms "contain", "comprise", and "include" used herein are synonymous, inclusive or open-ended, and do not exclude additional, unrecited members, elements, or method steps.

Numerical ranges expressed by endpoints in the present disclosure include all numbers and fractions included within the range, as well as the recited endpoints.

The present disclosure relates to a concentration value, and the fluctuations of the value are within a certain range. For example, it can fluctuate within the corresponding precision range. For example, with regard to the value 2%, fluctuations within the range of ± 0.1% may be permitted. For larger values or values that do not require way too fine control, the meaning is also allowed to include larger fluctuations. For example, with regard to the value 100 mM, fluctuations within the range of ± 1%, ± 2%, ± 5%, etc. may be permitted.

In the present disclosure, expressions involving terms such as "a plurality of" and "multiple" refer to a quantity greater than or equal to 2, unless otherwise specified.

In the present disclosure, "first" and "second" are only used to distinguish promoters that regulate different genes, but they do not have any definitive effect on the types, quantities, etc. of promoters.

In the present disclosure, the technical features described in an open-ended manner include both a closed technical solution consisting of the listed features and an open-ended technical solution comprising the listed features.

In the present disclosure, the expressions "preferably", "preferentially", "more preferably", and "appropriately" are solely used for describing better embodiments or examples, and it should be understood that the scope of the present disclosure is not intended to be limited. In the present disclosure, the expressions "optionally", "optional" and "alternative" mean that the subject modified by the expressions are dispensable, that is, the expressions mean that the parallel technical solutions "with" or "without" the subject modified by the expressions can both be selected. If the expression "alternative" appears multiple times in a technical solution, unless otherwise specified and without any contradictions or mutually restrictive relationships, the expression "alternative" is independent in each occurrence.

The present disclosure relates to an adeno-associated virus structural plasmid, which is provided with a Rep gene expression cassette and a Cap gene expression cassette in sequence in the gene expression direction, wherein
the Rep gene expression cassette comprises a first promoter and a *rep* gene regulated thereby for transcription, followed by a transcription termination signal fragment arranged downstream of the *rep* gene; and the Cap gene expression cassette comprises a second promoter and a *cap* gene regulated thereby for transcription,
wherein the first promoter comprises any one of a P5 promoter, an RSV promoter, an MMTV promoter, a UBC promoter, and a U6 promoter, and the second promoter comprises one or more of a CMV promoter, a CBh promoter, a CAG promoter, an EF1α promoter, and an SFFV promoter.

The inventors of the present disclosure have unexpectedly found that due to the ITR-based self-replication of DNAs, a higher yield of virus particles per cell may be related to (a) a higher copy number of *cap* gene per cell, and (b) the down-regulated expression of Rep protein level which has a positive impact on vector yield and improves Rep-mediated cytotoxicity that otherwise would affect cell viability.

In the present disclosure, relevant elements such as the promoter and codons in the adeno-associated virus structural plasmid are modified by this method, thereby achieving the effect of producing an adeno-associated virus helper vector with a high titer and a high infectious activity. The modified plasmid can be used in production of an adeno-associated virus vector by transient transfection of cells in suspension to meet the need of commercial use in the later stage. By establishing a stable large-scale suspension cell production process platform, a virus vector, which has a high titer and a high purity and is aseptic and pyrogen-free throughout the preparation process, scalable, economical, and controllable in quality, can be produced, thus improving the quality of the adeno-associated virus product in the whole industry.

Based on the above content, the first promoter may be any weak promoter of a P5 promoter, an RSV promoter, an MMTV promoter, a UBC promoter, or a U6 promoter. In some embodiments, the first promoter is a P5 promoter. The P5 promoter may be either intrinsically included in the adeno-associated virus structural plasmid or subsequently incorporated. The second promoter may be a single strong promoter, such as a CMV promoter, a CBh promoter, a CAG promoter, an EF1α promoter, an SFFV promoter or the like; may also contain two or more promoters, such as a CMV promoter and an EF1α promoter; or may also be a promoter with other genes that regulate transcription initiation, such as a CMV promoter and a CMV enhancer.

In some embodiments, in the Rep gene expression cassette, the translation initiation codon of the *rep* gene is an initiation codon different from ATG. It is easy to understand that the optimal translation initiation codon ATG is replaced with some initiation codons that are not conducive to translation, thus weakening gene expression. In some embodiments, the initiation codons of *rep78* and *rep68* are replaced with initiation codons different from ATG. In some embodiments, the initiation codons of *rep78, rep68, rep52,* and *rep40* are all replaced with initiation codons different from ATG. In a more preferred embodiment, the initiation codon is selected from ACG, CAG, TTG, ATT, CTG, or GTG codon. The codon here is a codon corresponding to the DNA codon table.

In some embodiments, the Cap gene expression cassette further comprises a P40 promoter. The P40 promoter and the second promoter co-regulate the transcription of *cap* gene.

In some embodiments, in the Cap gene expression cassette, a transcription termination signal fragment is provided downstream of the *cap* gene.

The transcription termination signal can function to achieve the purpose of terminating the transcription of related genes. The transcription termination signal may be either a single copy or multiple copies, e.g., 3×SV40 polyA, or may also be a combination of many different polyAs. In some embodiments, the transcription termination signal fragments in the Rep gene expression cassette and the Cap gene expression cassette are independently selected from one or more of SV40 polyA, BGH polyA, TK polyA, hGH polyA, or rbGlob polyA. The sequences of the above transcription termination signal fragments and promoters are well known to those skilled in the art, and the mutants thereof in a moderate range, e.g., with truncation, insertion, substitution and/or deletion of one or more nucleotides, are also encompassed. For example, the concept of BGH polyA encompasses short BGH polyA.

In the present disclosure, the *"rep* gene" in the Rep gene expression cassette comprises at least *rep78* and *rep68* genes and preferably comprises *rep52* and *rep40* genes. The *rep52* and *rep40* genes in the *rep* gene can be expressed by different plasmids.

In some preferred embodiments, the Rep gene expression cassette further comprises a *rep* gene (preferably *rep52* and *rep40* genes), regulated by a P19 promoter for transcription. Preferably, the expression regions of *rep52* and *rep40* genes are located downstream of *rep78* and *rep68* genes, and the *rep78* and *rep68* genes are regulated by the first promoter.

In some specific embodiments, the nucleotide sequence of the P5 promoter is as set forth in SEQ ID NO: 1; or a nucleotide sequence that has at least 80% identity thereto and can exert a transcription function not higher than that of the P5 promoter, e.g., a nucleotide sequence that has at least 85%, 90%, 95%, 96%, 97%, 98%, and 99% identity.

In some specific embodiments, the nucleotide sequence of the P40 promoter is as set forth in SEQ ID NO: 2; or a nucleotide sequence that has at least 80% identity thereto and can exert transcription function, e.g., a nucleotide sequence that has at least 85%, 90%, 95%, 96%, 97%, 98%, and 99% identity.

In some specific embodiments, the nucleotide sequence of the CMV promoter is as set forth in SEQ ID NO: 3; or a nucleotide sequence that has at least 80% identity thereto and can exert transcription function, e.g., a nucleotide sequence that has at least 85%, 90%, 95%, 96%, 97%, 98%, and 99% identity.

In some specific embodiments, the nucleotide sequence of SV40 polyA is as set forth in SEQ ID NO: 4; or a nucleotide sequence that has at least 80% identity thereto and can exert termination function, e.g., a nucleotide sequence that has at least 85%, 90%, 95%, 96%, 97%, 98%, and 99% identity.

In some specific embodiments, the nucleotide sequence of BGH polyA is as set forth in SEQ ID NO: 5; or a nucleotide sequence that has at least 80% identity thereto and can exert termination function, e.g., a nucleotide sequence that has at least 85%, 90%, 95%, 96%, 97%, 98%, and 99% identity.

In some specific embodiments, the nucleotide sequence of the P19 promoter is as set forth in SEQ ID NO: 6; or a nucleotide sequence that has at least 80% identity thereto and can exert transcription function, e.g., a nucleotide sequence that has at least 85%, 90%, 95%, 96%, 97%, 98%, and 99% identity.

In some specific embodiments, the nucleotide sequence of the CBh promoter is as set forth in SEQ ID NO: 8; or a nucleotide sequence that has at least 80% identity thereto and can exert transcription function, e.g., a nucleotide sequence that has at least 85%, 90%, 95%, 96%, 97%, 98%, and 99% identity.

In some specific embodiments, the nucleotide sequence of the CAG promoter is as set forth in SEQ ID NO: 9; or a nucleotide sequence that has at least 80% identity thereto and can exert transcription function, e.g., a nucleotide sequence that has at least 85%, 90%, 95%, 96%, 97%, 98%, and 99% identity.

In some specific embodiments, the nucleotide sequence of the EF1α promoter is as set forth in SEQ ID NO: 10; or a nucleotide sequence that has at least 80% identity thereto and can exert transcription function, e.g., a nucleotide sequence that has at least 85%, 90%, 95%, 96%, 97%, 98%, and 99% identity.

In some embodiments, the adeno-associated virus structural plasmid is derived from pAAV2/2 plasmid, and an original P40 promoter of the pAAV2/2 plasmid is inactivated or deleted. The pAAV2/2 plasmid refers to a plasmid corresponding to Cat. No. 104963 from Addgene. As used herein, the term "derived from" refers to the source on which modification is performed, that is, the cloning vector is obtained by modifying the initial vector from which it is derived (e.g., pAAV2/2 plasmid), and the modification may comprise: (i) inserting one or more exogenous promoters into an initial vector such as pAAV2/2 plasmid; (ii) inserting one or more transcription termination signal fragments into the initial vector such as pAAV2/2 plasmid; (iii) performing mutation on the initial vector such as pAAV2/2 plasmid to reduce the expression of *rep,* typically, for example, mutating the translation initiation codon ATC of *rep* into an initiation codon unfavorable for translation; (iv) performing mutation on the initial vector such as pAAV2/2 plasmid to inactivate unnecessary additional promoters; or combinations of (i) to (iv).

In some embodiments, the P40 promoter contained in the *rep* gene of the adeno-associated virus structural plasmid is inactivated or deleted.

In some specific embodiments, the P40 promoter contained in the *rep* gene of the adeno-associated virus structural plasmid is mutated into the DelP40 promoter; In some embodiments, the nucleotide sequence of the DelP40 promoter is as set forth in SEQ ID NO: 7 or a nucleotide sequence that has at least 80% identity thereto and can exert transcription function, e.g., a nucleotide sequence that has at least 85%, 90%, 95%, 96%, 97%, 98%, and 99% identity.

According to yet another aspect, the present disclosure further relates to a kit comprising the adeno-associated virus structural plasmid as described above.

In some embodiments, the kit further comprises a transfer plasmid and/or a helper plasmid.

The "transfer plasmid" in the present disclosure refers to a plasmid for carrying a target gene, which usually contains ITR sequences. The "target gene" refers to a gene derived from an entity, the genotype of which is distinct from those of other entities being compared therewith. For instance, a polynucleotide introduced into a plasmid or vector derived from a different species by genetic engineering technology is a heterologous polynucleotide. The target gene may include, for example, a gene (nucleotide sequence) encoding a protein that is defective or absent in a recipient individual or target cell; a gene that encodes a protein having a desired biological or therapeutic effect (such as antibacterial, antiviral, or antitumor/anticancer function); a nucleotide sequence encoding an RNA that inhibits or reduces the production of a deleterious or otherwise undesirable protein (for example, a nucleotide sequence encoding an RNA interfering agent as defined above); and/or a nucleotide sequence encoding an antigenic protein. In some embodiments, the expression product of the target gene is an interfering RNA or an aptamer. The interfering RNA can be selected from, for example, an siRNA, an shRNA, or an miRNA. In some embodiments, the expression product of the target gene is an mRNA, and such an mRNA is preferably capable of expressing a protein, such as a protein that endows the target cell with certain required characteristics, e.g., a fluorescent protein that allows for cell tracing, an active enzyme provided to compensate for losses or alterations in the target cell, and an antigen substance used as a vaccine. In some embodiments, the expression product of the target gene is a chimeric antigen receptor.

Suitable target genes include, but are not limited to, those that encode at least one of the following various proteins: interferons (e.g., IFN-γ, IFN-α, IFN-β, IFN-ω, and IFN-τ); insulin (e.g., Novolin, Humulin, Humalog, Humalog, and Lantus); erythropoietin ("EPO", e.g., epoetin-α, darbepoetin-α, and epoetin-β); antibodies (e.g., monoclonal antibodies, such as rituximab, infliximab, trastuzumab, HumiraTM (adalimumab), omalizumab, tositumomab, RaptivaTM (efalizumab), ErbituxTM (cetuximab), and bevacizumab); antigen-binding fragments, including monoclonal antibodies (e.g., ranibizumab); blood factors (e.g., alteplase, tissue plasminogen activator protein, recombinant human factor VIIa, factor VIIa, factor VIII, factor IX, β-globulin, and hemoglobin); colony stimulating factors (e.g., filgrastim (G-CSF), Neulasta (PEGylated filgrastim), granulocyte colony stimulating factor (G-CSF), granulocyte monocyte colony stimulating factor, macrophage colony stimulating factor, and megakaryocyte colony stimulating factor); growth hormone (e.g., somatotropin and growth hormone); interleukin (e.g., IL-1, IL-2, IL-3, IL-4, IL-5, IL-6, IL-7, IL-8, and IL-9); growth factors (e.g., beclapermin (PDGF), trafermin (bFGF), ancestim (stem cell factor), keratinocyte growth factor, acidic fibroblast growth factor, stem cell factor, basic fibroblast growth factor, and hepatocyte growth factor); soluble receptors (e.g., soluble TNF-α-binding receptors, such as etanercept, soluble VEGF receptors, soluble interleukin receptors, and soluble γ/δT cell receptors); enzymes (e.g., α-glucosidase, β-glucocerebrosidase, and alglucerase), and enzyme activators (e.g., tissue plasminogen activator); chemokines (e.g., IP-10, Mig, Groα/IL-8, RANTES, MIP-1α, MIP-1β, MCP-1, and PF-4); angiogenic agents (e.g., vascular endothelial growth factor (VEGF)); antiangiogenic agents (e.g., soluble VEGF receptors); protein vaccines; neuroactive peptides, such as bradykinin, cholecystokinin, gastrin, secretin, oxytocin, gonadotropin-releasing hormone, β-endorphin, enkephalin, substance P, growth hormone release inhibitory factor, prolactin, galanin, growth hormone releasing hormone, bombesin, dynorphin, neurotensin, motilin, thyrotropin, neuropeptide Y, luteinizing hormone, calcitonin, insulin, glucagon, vasopressin, angiotensin II, thyrotropin releasing hormone, vasoactive intestinal peptide, and sleep-inducing peptide; other proteins, e.g., thrombolytic agents, atrial natriuretic peptide, bone morphogenetic protein, thrombopoietin, relaxin, glial fibrillary acidic protein, follicle-stimulating hormone, human α-1 antitrypsin, leukemia inhibitory factor, transforming growth factor, insulin-like growth factor, luteinizing hormone, macrophage activating factor, tumor necrosis factor, neutrophil chemotactic factor, nerve growth factor, and tissue inhibitors of metalloproteinases; vasoactive intestinal peptide, angiopoietin, angiopoietin, and fibrin; hirudin; leukemia inhibitory factor; IL-1 receptor antagonists (e.g., anakinra); ion channels, e.g., cystic fibrosis transmembrane conductance regulator protein (CFTR); dystrophin; utrophin (a tumor inhibitor); lysosomal acid α-glucosidase (GAA); *etc.* Suitable target genes also include those encoding functional fragments of any of the above proteins; and nucleic acids encoding functional variants of any of the above proteins.

The "helper plasmid", also referred to as Helper plasmid, in the present disclosure refers to a plasmid containing relevant genes (such as E2A, E4, and VARNA) that mediate the replication of adeno-associated viruses. A variety of such helper viruses of AAV are known in the art, including, for example, adenovirus, herpesvirus, and poxvirus (such as vaccinia). Many adenoviruses derived from human, non-human mammals, and birds are known and can be obtained from collection centers such as CCTCC and CGMCC. Viruses of the herpes virus family include, for example, herpes simplex virus (HSV) and Epstein-Barr viruses (EBV) as well as cytomegalovirus (CMV) and pseudorabies virus (PRV).

The above adeno-associated virus structural plasmids, transfer plasmids, or helper plasmids may also comprise selectable markers (such as selectable genes; or tags that facilitate enrichment, e.g., his tag; or tags that facilitate detection, such as GFP), and sequences that enable autonomous replication of the DNA construct in prokaryotic or eukaryotic host cells, e.g., origin of replication (ori), or regulatory elements that are necessary for affecting the expression in specified target cells.

In some embodiments, the above plasmid comprises two or more, for example, 3, 4, 5, 6, 7, 8, 9, 10 or more endonuclease recognition sequences.

In some embodiments, the above plasmid comprises a selectable gene, which is, for example, typically an antibiotic resistance gene, such as kanamycin resistance gene (*nptII*), tetracycline resistance gene (*tetR*), or neomycin phosphotransferase gene (*npt*). The selectable gene may also be a gene of an enzyme for compound detoxification, a selectable marker gene for carbohydrate-metabolizing enzyme, etc.

The genome of the adeno-associated virus particles as claimed by the present disclosure may be derived from any AAV virus. The adeno-associated virus particle may also be used interchangeably with "AAV virus", "AAV virus particle" or "rAAV vector particle". "AAV" is the abbreviation of adeno-associated virus and can be used to refer to the virus itself or a derivative thereof. Unless otherwise required, the terms include subtypes and naturally occurring and recombinant forms. The abbreviation "rAAV" refers to recombinant adeno-associated virus, also known as recombinant AAV vector (or "rAAV" vector). The term "AAV" can be used to refer to various serotypes of adeno-associated virus. To date, numerous serotypes of AAV have been identified, including 13 human serotypes and more than 100 non-human primate serotypes. Examples are type 1 AAV (AAV1), type 2 AAV (AAV2), type 3 AAV (AAV3), type 4 AAV (AAV4), type 5 AAV (AAV5), type 6 AAV (AAV6), type 7 AAV (AAV7), type 8 AAV (AAV8), type 9 AAV (AAV9), type 10 AAV (AAV10), type 11 AAV (AAV11), type 12 AAV (AAV12), type 13 AAV (AAV13), avian AAV, bovine AAV, canine AAV, equine AAV, primate AAV, non-primate AAV, and ovine AAV. "Primate AAV" refers to AAV that infects primates, "non-primate AAV" refers to AAV that infects non-primate mammals, "bovine AAV" refers to AAV that infects bovine mammals, and so on. Among them, AAV2, AAV3, and AAV9 are derived from human beings themselves and are thus preferred; and AAV2 is more preferred. Genomic sequences of different subtypes of AAV, as well as natural terminal repeats (ITRs), Rep protein and capsid subunit sequences, are known in the art. Such sequences can be found in literatures or public databases such as GenBank.

The *rep* gene and *cap* gene in the corresponding serotype used by the present disclosure may be either wild-type or mutant in a moderate range, and other technologies, such as rational mutagenesis, targeting peptide engineering, generation of chimeric particles, library and directed evolution method, and immune escape modification, are used to optimize the sequence of the *rep* gene and *cap* gene sequences in the AAV vector, for example, to achieve the purpose of immune escape and enhancing therapeutic output. Reference can be made to, for example, Mitchell A.M. et al., AAV's anatomy: Roadmap for optimizing vectors for translational success. Curr Gene Ther. 10(5): 319-340.

The kit provided by the present disclosure can further comprise some conventional components, for example, restriction endonucleases matched with the restriction sites contained in the above plasmid, and also for example, instructions that records the method for using the kit.

The present disclosure further relates to a method for preparing adeno-associated virus particles, comprising introducing the adeno-associated virus structural plasmid as described above, together with a transfer plasmid and a helper plasmid, into a host cell for packaging and culture, lysing the host cell, and then collecting the adeno-associated virus particles.

Any method known in the art can be used to introduce the above plasmid into a host cell, and the method includes, but is not limited to, chemical transformation or electroporation transformation.

In some embodiments, the host cell can provide adenovirus E1 regions (e.g., E1a and E1b). The host cell can be a mammalian (e.g., murine, rabbit, canine, monkey, bovine, ovine, horse, donkey, and human) cell or cell line that is common in the art.

In some embodiments, the host cell is an HEK-293 cell or a derivative strain thereof. In some specific embodiments, the host cell is a suspended HEK293 cell. In one embodiment, the host cell is a PowerS^{™}-293 cell.

In some embodiments, the derivative strain is selected from at least one of HEK-293A, HEK-293S, HEK-293SG, HEK-293SGGD, HEK-293T, HEK-293T/17 SF, HEK-293H, HEK-293E, HEK-293-6E, HEK-293F, HEK-293FT, HEK-293FTM, AAV-293, and GP2-293.

In some embodiments, the plasmid is introduced into the host cell by a transient transfection method. In some other embodiments, the host cell is cultured in suspension.

Optionally, the method further comprises a step of further separating and purifying the adeno-associated virus particles.

The embodiments of the present disclosure will be described in detail below with reference to the examples. It should be understood that these examples are merely intended to illustrate the present disclosure but not to limit the scope of the present disclosure. The experimental methods in the following examples, where no specific conditions are specified, can be performed preferentially according to the instructions provided in the present disclosure, experimental manual or conventional conditions in the art, other experimental methods known in the art, or the conditions suggested by the manufacturer.

In the following specific examples, the measurement parameters related to the components of the raw materials may have slight deviations within the weighing precision, unless otherwise specified. When temperature and time parameters are involved, acceptable deviations due to instrument testing precision or operating precision are allowed.

### Example 1 Design, synthesis and verification of pAAV-Rep2/Cap2 plasmid

Based on the autonomously synthesized pAAV-Rep2/Cap2 plasmid (hereafter collectively referred to as pRC2 in short) plasmid (reference is made to pAAV2/2 plasmid from Addgene, Cat. No.: 104963), which comprises elements such as *rep* gene, *cap* gene, P19 and P40 promoters, the following two directions were selected for modification:
1. reducing the expression of *rep*: by adding the P5 promoter and changing ATG codon to a weaker ACG codon;
2. enhancing the expression of *cap*: under the premise of not affecting the encoded amino acids, the P40 promoter in *rep* gene was mutated such that it loses its function (that is, P40 was mutated into DelP40), and polyA tail was added after *rep* gene; in addition, the expression of Cap gene could be regulated and enhanced independently by adding the CMV promoter before *cap* gene, and the expression of Cap gene was then further enhanced by CMV+P40 dual promoters.

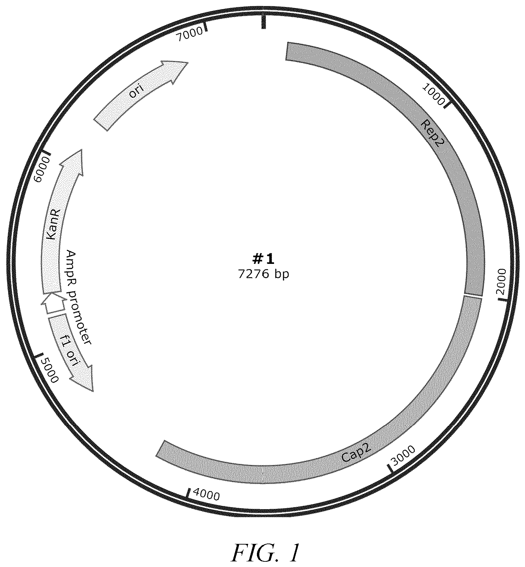

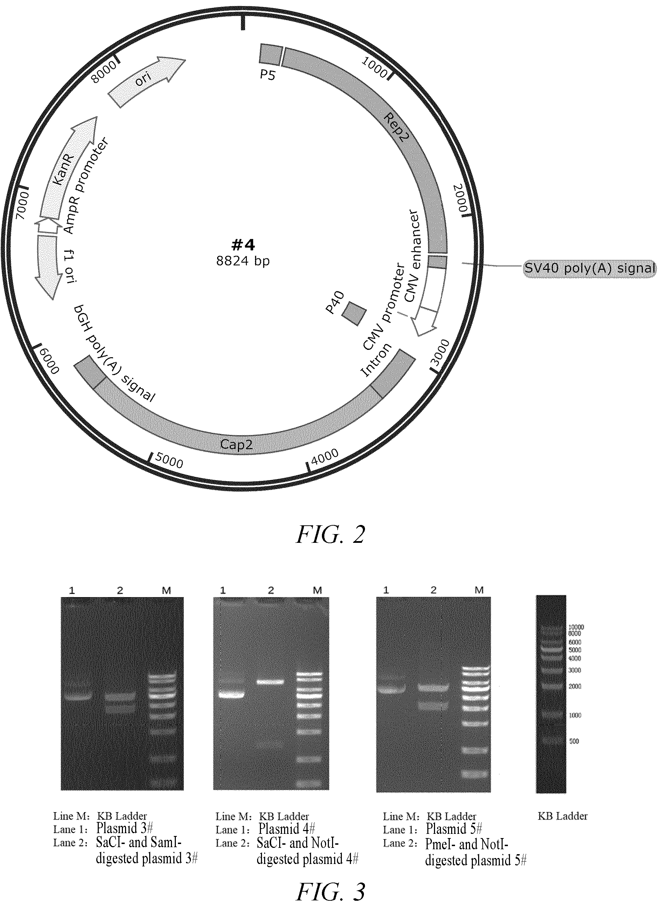

According to the above modification concept, the following five modified pRC2 plasmid sequences (plasmids 2#-6#) were designed in order to achieve the purpose of improving virus titer, wherein plasmid 1# is the original unmodified plasmid (see FIG. 1), and a map of the representative modified plasmid 4# is shown in FIG. 2.
1#: pRC2-ATG-Rep(P40)-Cap
2#: pRC2-MMTV-ACG-Rep(DelP40)-SV40polyA-CMV-Cap-BGHpolyA;
3#: pRC2-P5-ACG-Rep(DelP40)-SV40polyA-CMV-Cap-BGHpolyA;
4#: pRC2-P5-ACG-Rep(DelP40)-SV40polyA-CMV-P40-Cap-BGHpolyA;
5#: pRC2-P5-ATG-Rep(DelP40)-SV40polyA-CMV-P40-Cap-BGHpolyA;
6#: pRC2-P5-ACG-Rep(DelP40)-SV40polyA-P40-Cap-BGHpolyA.

The sequences of the above elements are as shown below:
P5 promoter: 
GGTGGAGTCGTGACGTGAATTACGTCATAGGGTTAG
GGAGGTCCTGTATTAGAGGTCACGTGAGTGTTTTGCGACATTTTGCGAC
 
ACCATGTGGTCACGCTGGGTATTTAAGCCCGAGTGAGCACGCAGGGTC
TCCATTTTGAAGCGG (SEQ ID NO: 1)
P19 promoter: 
GCCGGAGGCGGGAACAAGGTGGTGGATGAGTGCTACATCCCCAAT
TACTTGCTCCCCAAAACCCAGCCTGAGCTCCAGTGGGCGTGGACTAATA
TGGAACAGTATTTAAGCGCCTGTTTGAATCTCACGGAGCGTAAACGGTT
GGTGGCG (SEQ ID NO: 6)
DelP40 promoter: 
GGTCACCAAGCAAGAGGTTAAGGACTTTTTCCGGTGGGCAAAGGA
TCACGTGGTTGAGGTGGAGCATGAATTCTACGTCAAAAAGGGTGGAGC
CAAGAAAAGACCAGCACCAAGTGACGCAGACATCAGCGAGCCCAAAC
GGGTGCGCGAGTCAGTTGCGCAGCCATCGACGTCAGACGC (SEQ ID NO:
7)
 
P40 promoter: GGTCACCAAGCAGGAAGTCAAAGACTTTTTCCGGTG
GGCAAAGGATCACGTGGTTGAGGTGGAGCATGAATTCTACGTCAAAAA
GGGTGGAGCCAAGAAAAGACCCGCCCCCAGTGACGCAGATATAAGTG
AGCCCAAACGGGTGCGCGAGTCAGTTGCGCAGCCATCGACGTCAGACG
C (SEQ ID NO: 2)
CMV promoter: 
CGTTACATAACTTACGGTAAATGGCCCGCCTGGCTGACCGCCCAAC
GACCCCCGCCCATTGACGTCAATAATGACGTATGTTCCCATAGTAACGC
CAATAGGGACTTTCCATTGACGTCAATGGGTGGAGTATTTACGGTAAAC
TGCCCACTTGGCAGTACATCAAGTGTATCATATGCCAAGTACGCCCCCT
ATTGACGTCAATGACGGTAAATGGCCCGCCTGGCATTATGCCCAGTAC
ATGACCTTATGGGACTTTCCTACTTGGCAGTACATCTACGTATTAGTCA
TCGCTATTACCATGCTGATGCGGTTTTGGCAGTACATCAATGGGCGTGG
ATAGCGGTTTGACTCACGGGGATTTCCAAGTCTCCACCCCATTGACGTC
AATGGGAGTTTGTTTTGGCACCAAAATCAACGGGACTTTCCAAAATGTC
GTAACAACTCCGCCCCATTGACGCAAATGGGCGGTAGGCGTGTACGGT
GGGAGGTCTATATAAGCAGAGCT (SEQ ID NO: 3)
SV40 poly(A): 
AACTTGTTTATTGCAGCTTATAATGGTTACAAATAAAGCAATAGCA
TCACAAATTTCACAAATAAAGCATTTTTTTCACTGC (SEQ ID NO: 4)
BGH poly(A):

CTGTGCCTTCTAGTTGCCAGCCATCTGTTGTTTGCCCCTCCCCCGTG
CCTTCCTTGACCCTGGAAGGTGCCACTCCCACTGTCCTTTCCTAATAAA
ATGAGGAAATTGCATCGCATTGTCTGAGTAGGTGTCATTCTATTCTGGG
GGGTGGGGTGGGGCAGGACAGCAAGGGGGAGGATTGGGAAGACAATA
GCAGGCATGCTGGGGA (SEQ ID NO: 5)

These five plasmids were synthesized by GenScript (Nanjing) Co., Ltd. on commission. Enzymatic digestion identification and Sanger sequencing were carried out. Enzymatic digestion and electrophoresis conditions were as follows: according to different plasmids, 500 ng of a plasmid was pipetted, and double enzymatic digestion was carried out by adding different enzymes, such as SacI, NotI, PmeI, and SmaI, followed by incubation at 37°C for 60 min. Subsequently, an electrophoresis test was carried out on 1% agarose gel at a set voltage of 100 V for 30 min. The analytic results, as shown in FIG. 3, verified that the sizes of fragments resulting from the enzymatic digestion of the modified plasmids 3#, 4#, and 5# were consistent with the theoretical fragment sizes, and Sanger sequencing verified the correctness of the sequences.

### Example 2 Preparation and verification of pAAV-Rep2/Cap2 plasmid

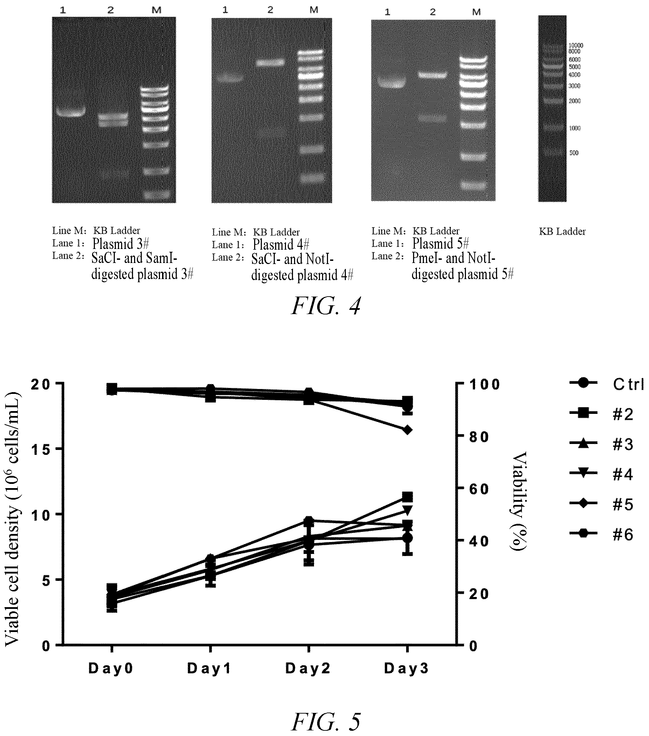

Plasmids were prepared by NEB Stable strain (purchased from NEW ENGLAND BioLabs, Cat. No.: C3040H), with the process including plasmid transformation and verification. The steps were as follows:
1. Competent Stable cells were taken and placed in an ice bath.
2. The above six plasmid sequences were respectively added to competent Stable cell suspensions, flicked gently and mixed uniformly, and left to stand in an ice bath for 30 min.
3. Centrifuge tubes were placed in a water bath at 42°C for 60-90 seconds, and the tubes were then quickly transferred to an ice bath to cool the competent Stable cells for 2-3 min, without shaking the centrifuge tube during the process.
4. 900 µl of a sterile LB medium (without antibiotics) was added to the centrifuge tubes and mixed uniformly, and the centrifuge tubes were then placed in a shaker at 37°C for shaking culturing for 45-60 min to resuscitate the bacteria.
5. The centrifugal tubes were mixed uniformly, and 100 µl of transformed competent Stable cells were pipetted and added to an LB solid agar medium plate containing a corresponding antibiotic and then spread evenly with a sterile spreader. The plate was placed at room temperature until the liquid was completely absorbed, and the plate was inverted and cultured at 37°C for 12-16 hours.
6. Plaques were picked from the plate, in which colonies were grown, and placed in an LB medium, which was placed into a shaker at 37°C for shaking culture, and the bacteria were preserved in glycerol.
7. Plasmids were extracted with a kit (purchased from QIAGEN, Cat. No.: 12391), and the plasmids were identified by enzymatic digestion and agarose gel electrophoresis. The identification gel diagrams of plasmids 3#, 4#, and 5# were as shown in FIG. 4, in which the sizes of fragments resulting from the enzymatic digestion of plasmids 3#, 4#, and 5# were consistent with the theoretical fragment sizes. The plasmids were subjected to Sanger sequencing to verify the correctness of the sequence results.

Enzymatic digestion and electrophoresis conditions: according to different plasmids, 500 ng of a plasmid was pipetted, and double enzymatic digestion was carried out by adding different enzymes, such as SacI, NotI, PmeI, and SmaI, followed by incubation at 37°C for 60 min. Subsequently, an electrophoresis test was carried out on 1% agarose gel at a set voltage of 100 V for 30 min.

### Plasmid fermentation:

The bacteria in glycerol obtained in the last step were inoculated into an LB medium at a proportion of 1-2% and placed into a shaker at 37°C for shaking culture for 16-18 h. Plasmids with a final concentration of 1 mg/mL and without endotoxin was prepared by means of a large-scale plasmid extraction kit, for later use.

### Example 3 Verification of effect of plasmids on adeno-associated virus titer

1. PowerS^{™}-293 cells (GenScript) were resuscitated in advance, passaged at 0.5E+6 cells/mL, and cultured in an 8% CO₂ incubator at a constant temperature of 37°C at 125 rpm for 3 days.
2. With PowerS^{™}-293 cell density ≥ 4E+6 cells/mL and viability ≥ 90%, pHelper plasmid (reference is made to pAdDeltaF6 from Addgene, Cat. No.: 112867), the above modified pAAV-Rep2/Cap2 plasmid and pAAV-GFP plasmid (reference is made to AAV GFP plasmid from Addgene, Cat. No.: 49055) were used. The amount of the plasmids added was 0.375 µg/10⁶ cells, and the plasmids were mixed at a ratio of 1 : 3 : 1. The AAV2 virus vector was packaged by means of a three-plasmid transient transfection method.
3. The viable cell density and viability were detected by Vi-CELL XR fully automatic cell counting and viability analysis system (purchased from Beckman Coulter, Cat. No.: VICELL-XR) every day within three days after transfection of cells. As shown in FIG. 5, the viability of transfected cells was comparable to that in the case of plasmid 1# in the control group, or the cell density was even higher after transfection with the modified plasmid.
4. The virus was harvested 72 h after transfection, and a lysis solution was added to the cultured cells at a ratio of lysis solution (Tween20, purchased from Sigma-Aldrich, Cat. No.: 9005-64-5) : cell suspension = 1 : 9 (volume ratio). The mixture was incubated at 37°C for 1 h, centrifuged at 3000 g at 4°C for 20 min, and the supernatant was taken and aliquoted.

The genome copy number was detected according to the operation steps in the instructions of AAVpro^{®} Titration Kit (for real-time PCR, TAKARA, Cat. No.: 6233). AAV functional titer (transduction titer) was obtained as follows: AAV virus was diluted in gradient and then was allowed to infect HEK293T adherent cells; the expression of GFP gene was detected by a flow cytometer to determine whether it was positive for GFP; and according to the number of positive cells or positive wells, combined with virus dilution, the transduction titer was calculated, with its measurement unit being Transduction unit (TU).

**Table 1 Statistical table of genome copy number data**

| Plasmid No. | Average value of three genome copy number measurements (vg/mL) | Standard deviation | CV |
|---|---|---|---|
| Ctrl | 2.49E+10 | 4.91E+09 | 0.20 |
| #2 | 1.06E+10 | 1.45E+09 | 0.14 |
| #3 | 6.98E+10 | 6.15E+09 | 0.09 |
| #4 | 1.05E+11 | 7.93E+09 | 0.08 |
| #5 | 1.01E+11 | 2.42E+10 | 0.24 |
| #6 | 3.86E+10 | 6.96E+09 | 0.18 |

**Table 2 Statistical table of transduction titer data**

| Plasmid No. | Average value of three activity titer measurements (TU/mL) | Standard deviation | CV |
|---|---|---|---|
| Ctrl | 3.53E+06 | 5.27E+05 | 0.15 |
| #2 | 8.34E+05 | 8.02E+04 | 0.10 |
| #3 | 1.49E+07 | 1.77E+06 | 0.12 |
| #4 | 1.56E+07 | 1.22E+06 | 0.08 |
| #5 | 7.35E+06 | 1.28E+06 | 0.17 |
| #6 | 4.27E+06 | 6.64E+05 | 0.16 |

As shown in FIG. 5, there were differences in viable cell density and cell viability after transfection with the designed plasmids with different structures.

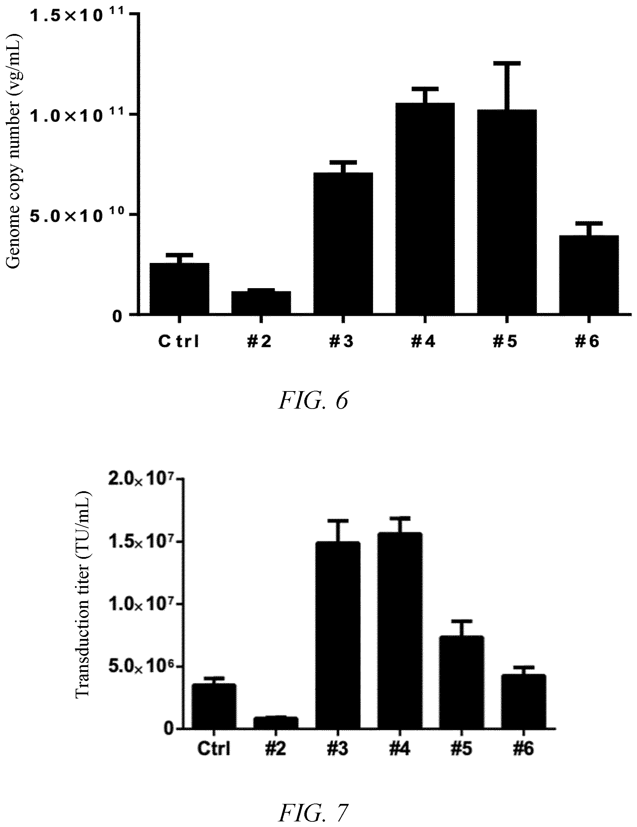

As shown in FIGs. 6 and 7 and Tables 1 and 2, in terms of genome copy number, compared with the control, plasmids #3, #4, and #5 showed improvements by 2.4, 4.2 and 4.1 folds, respectively, among which both plasmids #4 and #5 reached 1E+11 vg/mL; and in terms of transduction titer, compared with the control, plasmids #3, #4, and #5 showed improvements by 4.0, 4.1 and 2.0 folds, respectively, among which both plasmids #3 and #4 reached 1E+07 TU/mL.

### Example 4 Substitution with strong promoters in pAAV-Rep2/Cap2 and effect verification

In order to verify the universal effectiveness of strong promoters in improving the physical and biological titer of adeno-associated virus, the following three modified pRC2 plasmid sequences (plasmids 7#-9#) were designed with plasmid 4# as a template.
4#: pRC2-P5-ACG-Rep(DelP40)-SV40polyA-CMV-P40-Cap-BGHpolyA;
7#: pRC2-P5-ACG-Rep(DelP40)-SV40polyA-CBh-P40-Cap-BGHpolyA;
8#: pRC2-P5-ACG-Rep(DelP40)-SV40polyA-CAG-P40-Cap-BGHpolyA; and
9#: pRC2-P5-ACG-Rep(DelP40)-SV40polyA-EF1α-P40-Cap-BGHpolyA.

The sequences of the above corresponding strong promoter elements are as shown below:

CBh promoter: CGTTACATAACTTACGGTAAATGGCCCGCCTGGCTGA
CCGCCCAACGACCCCCGCCCATTGACGTCAATAGTAACGCCAATAGGG

ACTTTCCATTGACGTCAATGGGTGGAGTATTTACGGTAAACTGCCCACT
TGGCAGTACATCAAGTGTATCATATGCCAAGTACGCCCCCTATTGACGT
CAATGACGGTAAATGGCCCGCCTGGCATTGTGCCCAGTACATGACCTTA
TGGGACTTTCCTACTTGGCAGTACATCTACGTATTAGTCATCGCTATTA
CCATGGTCGAGGTGAGCCCCACGTTCTGCTTCACTCTCCCCATCTCCCC
CCCCTCCCCACCCCCAATTTTGTATTTATTTATTTTTTAATTATTTTGTGC
AGCGATGGGGGCGGGGGGGGGGGGGGGGCGCGCGCCAGGCGGGGCGG
GGCGGGGCGAGGGGCGGGGCGGGGCGAGGCGGAGAGGTGCGGCGGCA
GCCAATCAGAGCGGCGCGCTCCGAAAGTTTCCTTTTATGGCGAGGCGG
CGGCGGCGGCGGCCCTATAAAAAGCGAAGCGCGCGGCGGGCGGGAGT
CGCTGCGCGCTGCCTTCGCCCCGTGCCCCGCTCCGCCGCCGCCTCGCGC
CGCCCGCCCCGGCTCTGACTGACCGCGTTACTCCCACAGGTGAGCGGG
CGGGACGGCCCTTCTCCTCCGGGCTGTAATTAGCTGAGCAAGAGGTAA
GGGTTTAAGGGATGGTTGGTTGGTGGGGTATTAATGTTTAATTACCTGG
AGCACCTGCCTGAAATCACTTTTTTTCAG (SEQ ID NO: 8)

CAG promoter: CGTTACATAACTTACGGTAAATGGCCCGCCTGGCT
GACCGCCCAACGACCCCCGCCCATTGACGTCAATAATGACGTATGTTCC
CATAGTAACGCCAATAGGGACTTTCCATTGACGTCAATGGGTGGAGTA
TTTACGGTAAACTGCCCACTTGGCAGTACATCAAGTGTATCATATGCCA
AGTACGCCCCCTATTGACGTCAATGACGGTAAATGGCCCGCCTGGCATT
ATGCCCAGTACATGACCTTATGGGACTTTCCTACTTGGCAGTACATCTA
CGTATTAGTCATCGCTATTACCATGATGGTCGAGGTGAGCCCCACGTTC
TGCTTCACTCTCCCCATCTCCCCCCCCTCCCCACCCCCAATTTTGTATTT
ATTTATTTTTTAATTATTTTGTGCAGCGATGGGGGCGGGGGGGGGGGG
GGGGCGCGCGCAGGCGGGGCGGGGCGGGGCGAGGGGCGGGGCGGGGCG
AGGCGGAGAGGTGCGGCGGCAGCCAATCAGAGCGGCGCGCTCCGAAA
GTTTCCTTTTATGGCGAGGCGGCGGCGGCGGCGGCCCTATAAAAAGCG
AAGCGCGCGGCGGGCGGGAGTCGCTGCGCGCTGCCTTCGCCCCGTGCC
CCGCTCCGCCGCCGCCTCGCGCCGCCCGCCCCGGCTCTGACTGACCGCG
TTACTCCCACAGGTGAGCGGGCGGGACGGCCCTTCTCCTCCGGGCTGTA
ATTAGCGCTTGGTTTAATGACGGCTTGTTTCTTTTCTGTGGCTGCGTGAA
AGCCTTGAGGGGCTCCGGGAGGGCCCTTTGTGCGGGGGGAGCGGCTCG
GGGGGTGCGTGCGTGTGTGTGTGCGTGGGGAGCGCCGCGTGCGGCTCC
GCGCTGCCCGGCGGCTGTGAGCGCTGCGGGCGCGGCGCGGGGCTTTGT

GCGCTCCGCAGTGTGCGCGAGGGGAGCGCGGCCGGGGGCGGTGCCCCG
CGGTGCGGGGGGGGCTGCGAGGGGAACAAAGGCTGCGTGCGGGGTGT
GTGCGTGGGGGGGTGAGCAGGGGGTGTGGGCGCGTCGGTCGGGCTGCA
ACCCCCCCTGCACCCCCCTCCCCGAGTTGCTGAGCACGGCCCGGCTTCG
GGTGCGGGGCTCCGTACGGGGCGTGGCGCGGGGCTCGCCGTGCCGGGC
GGGGGGTGGCGGCAGGTGGGGGTGCCGGGCGGGGCGGGGCCGCCTCG
GGCCGGGGAGGGCTCGGGGGAGGGGCGCGGCGGCCCCCGGAGCGCCG
GCGGCTGTCGAGGCGCGGCGAGCCGCAGCCATTGCCTTTTATGGTAATC
GTGCGAGAGGGCGCAGGGACTTCCTTTGTCCCAAATCTGTGCGGAGCC
GAAATCTGGGAGGCGCCGCCGCACCCCCTCTAGCGGGCGCGGGGCGAA
GCGGTGCGGCGCCGGCAGGAAGGAAATGGGCGGGGAGGGCCTTCGTG
CGTCGCCGCGCCGCCGTCCCCTTCTCCCTCTCCAGCCTCGGGGCTGTCC
GCGGGGGGACGGCTGCCTTCGGGGGGGACGGGGCAGGGCGGGGTTCG
GCTTCTGGCGTGTGACCGGCGGCTCTAGAGCCTCTGCTAACCATGTTCA
TGCCTTCTTCTTTTTCCTACAG (SEQ ID NO: 9)

EF1α promoter: GGGCAGAGCGCACATCGCCCACAGTCCCCGAGAAG
TTGGGGGGAGGGGTCGGCAATTGAACCGGTGCCTAGAGAAGGTGGCGC
GGGGTAAACTGGGAAAGTGATGTCGTGTACTGGCTCCGCCTTTTTCCCG
AGGGTGGGGGAGAACCGTATATAAGTGCAGTAGTCGCCGTGAACGTTC
TTTTTCGCAACGGGTTTGCCGCCAGAACACAG (SEQ ID NO: 10)

These three plasmids were synthesized by GenScript (Nanjing) Co., Ltd. on commission. Enzymatic digestion identification and Sanger sequencing were carried out. The specific method was detailed in Example 1, in which the sizes of fragments resulting from the enzymatic digestion of modified plasmids 7#, 8#, and 9# were verified to be consistent with the theoretical fragment sizes, and Sanger sequencing verified the correctness of the sequences.

The specific method for plasmid preparation and verification was detailed in Example 2.

The specific method for verifying the effect of the strong promoters on adeno-associated virus titer was detailed in Example 3.

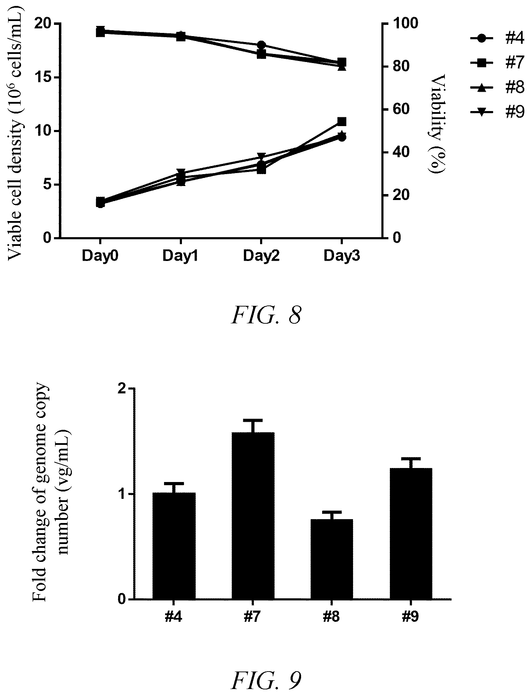

As shown in FIG. 8, in the case of only substitution with these strong promoters, the trends in cell density and viability after transfection kept consistent. On the third day after transfection, the viable cell density was approximately 1E+07 cells/mL, and the viable cell proportion was approximately 80%.

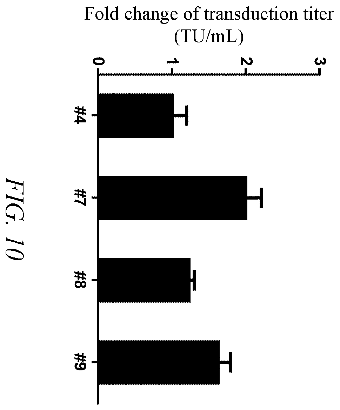

As shown in FIGs. 9 and 10, in terms of the genome copy number, plasmid #8 also showed a relatively great improvement compared with the control in the previous Example 3, plasmids #4, #7, and #9 all reached 1E+11 vg/mL, and compared with the optimal plasmid #4 in previous Example 3, plasmids #7 and #9 showed improvements by 1.5 and 1.2 folds, respectively, indicating further improved genome copy numbers. In terms of transduction titer, plasmids #4, #7, #8, and #9 all reached 1E+07 TU/mL, among them, plasmid #7 showed the largest improvement, and compared with the optimal plasmid #4 in the previous Example 3, plasmids #7, #8, and #9 showed improvements by 2.0, 1.2 and 1.6 folds, respectively, indicating better improved transduction titers. Generally, the modifications to plasmids #4, #7, #8, and #9 are successful, and the feasibility and universality of different strong promoters for titer improvement are verified.

At present, the genome copy number of AAV2 packaged on the platforms of major companies on the market is approximately 2-6E+10 vg/mL, and the transduction titer is approximately 2-6E+06 TU/mL which is lower than that of other serotypes. The low titer of AAV2 virus vector often leads to the failure of the downstream process to proceed normally, a low recovery rate, and a low infectious activity, and will have great side effects on the delivery efficiency of the virus vector. Based on these factors, the later commercial production will be greatly hindered. Most companies mainly improve AAV packaging titer by means of a series of process optimization means such as DoE screening and OFAT. However, these process optimization methods may result in drawbacks such as increasing plasmid residues and introducing other impurities. This not only increases the burden on downstream purification but also enhances the variability in subsequent process scale-up. Although there will be an effect of improving titer, the improvement extent is limited.

In the present disclosure, four pAAV-Rep/Cap plasmids of AAV2 are successfully screened out by substituting regulatory elements such as promoters and initiation codons by means of molecular biology. AAV2 packaging using the three-plasmid expression system platform process increases the genome copy number by 2-5 folds and the transduction titer by 3-5 folds, and also solves the problem of difficult downstream purification.

The embodiments of the present disclosure are not limited to the above-mentioned examples, and various modifications and improvements in forms and details may be made to the present disclosure by those of ordinary skill in the art without departing from the spirit and scope of the present disclosure, and the modifications and improvements are considered to fall within the scope of protection of the present disclosure.

The above examples merely represent several embodiments of the present disclosure, giving specifics and details thereof, but should not be understood as limiting the scope of the present patent of disclosure thereby. It should be noted that those of ordinary skill in the art could also make several alterations and improvements without departing from the spirit of the present disclosure and these would all fall within the scope of protection of the present disclosure. Therefore, the scope of protection of the present patent of disclosure shall be in accordance with the appended claims, and the description and accompanying drawings can be used to explain the content of the claims.

## Claims

1. An adeno-associated virus structural plasmid, which is provided with a Rep gene expression cassette and a Cap gene expression cassette in sequence in the gene expression direction, **characterized in that**
the Rep gene expression cassette comprises a first promoter and a *rep* gene regulated thereby for transcription, followed by a transcription termination signal fragment arranged downstream of the *rep* gene; and the Cap gene expression cassette comprises a second promoter and a *cap* gene regulated thereby for transcription,
wherein the first promoter comprises any one of a P5 promoter, an RSV promoter, an MMTV promoter, a UBC promoter, and a U6 promoter, and the second promoter comprises one or more of a CMV promoter, a CBh promoter, a CAG promoter, an EF1α promoter, and an SFFV promoter.

2. The adeno-associated virus structural plasmid according to claim 1, **characterized in that** in the Rep gene expression cassette, the translation initiation codon of the *rep* gene is an initiation codon different from ATG and is preferably selected from ACG, CAG, TTG, ATT, CTG, or GTG codon.

3. The adeno-associated virus structural plasmid according to claim 1, **characterized in that** the Cap gene expression cassette further comprises a P40 promoter.

4. The adeno-associated virus structural plasmid according to claim 1, **characterized in that** in the Cap gene expression cassette, a transcription termination signal fragment is provided downstream of the *cap* gene.

5. The adeno-associated virus structural plasmid according to claim 4, **characterized in that** the transcription termination signal fragments in the Rep gene expression cassette and the Cap gene expression cassette are independently selected from one or more of SV40 polyA, BGH polyA, TK polyA, hGH polyA, or rbGlob polyA.

6. The adeno-associated virus structural plasmid according to claim 1, **characterized in that** the Rep gene expression cassette further comprises a P19 promoter.

7. The adeno-associated virus structural plasmid according to any one of claims 1 to 6, **characterized in that** the adeno-associated virus structural plasmid is derived from pAAV2/2 plasmid, and an original P40 promoter of the pAAV2/2 plasmid is inactivated or deleted.

8. A kit, **characterized by** comprising the adeno-associated virus structural plasmid according to any one of claims 1 to 7.

9. The kit according to claim 8, **characterized by** further comprising a transfer plasmid and/or a helper plasmid.

10. The kit according to claim 8 or 9, **characterized in that** the kit is used for generating any one or more adeno-associated viruses of AAV1, AAV2, and AAV3-AAV13.

11. A method for preparing adeno-associated virus particles, **characterized by** comprising introducing the adeno-associated virus structural plasmid according to any one of claims 1 to 7, together with a transfer plasmid and/or a helper plasmid, into a host cell for packaging and culture, lysing the host cell, and then collecting the adeno-associated virus particles.

12. The method for preparing adeno-associated virus particles according to claim 11, **characterized in that** the host cell can provide adenovirus E1 region and is an HEK-293 cell or a derivative strain thereof.

13. The method for preparing adeno-associated virus particles according to claim 12, **characterized in that** the derivative strain is selected from at least one of HEK-293A, HEK-293S, HEK-293SG, HEK-293SGGD, HEK-293T, HEK-293T/17 SF, HEK-293H, HEK-293E, HEK-293-6E, HEK-293F, HEK-293FT, HEK-293FTM, AAV-293, and GP2-293.

14. The method for preparing adeno-associated virus particles according to any one of claims 11 to 13, **characterized in that** the plasmid is introduced into the host cell by a transient transfection method.

15. The method for preparing adeno-associated virus particles according to any one of claims 11 to 14, **characterized in that** the host cell is cultured in suspension.
